# FASCICULE DE BREVET EUROPEEN

(11) **EP 2 216 649 B1**
(45) Date de publication et mention de la délivrance du brevet: **21.10.2015**
(21) Numéro de dépôt: 10354006.8
(22) Date de dépôt: 02.02.2010
(51) Int. Cl.: G01N 33/487, G01N 27/333, G01N 27/403, G01N 27/416

(54) **Dispositif embarqué d'analyse d'un fluide corporel**
Tragbares Gerät zur Messung einer körperlichen Flüssigkeit
Portable device for analysing a bodily fluid

(30) Priorité: 06.02.2009 FR 0900526
(43) Date de publication de la demande: 11.08.2010
(73) Titulaire: Commissariat à l'Énergie Atomique et aux Énergies Alternatives, 75015 Paris (FR)
(72) Inventeur: Revol-Cavalier, Frédéric, 38180 Seyssins (FR)
(74) Mandataire: Dubreu, Sandrine

(56) Documents cités:
- WO-A-92/04630
- WO-A-99/35487
- US-A- 5 330 634
- US-B1- 6 650 933

## Description

### Domaine technique de l'invention

L'invention concerne un dispositif d'analyse d'un fluide corporel qui comporte:
- une cellule de mesure contenant le fluide corporel à analyser et munie d'au moins une électrode de travail de la cellule de mesure et d'au moins une électrode de référence de la cellule de mesure,
- au moins une cellule de référence contenant un étalon et munie d'au moins une électrode de travail de la cellule de référence et d'au moins une électrode de référence de la cellule de référence et,
- un circuit électronique de mesure connecté aux électrodes de travail et de référence, de la cellule de mesure et de la cellule de référence.

### État de la technique

De nombreux dispositifs embarqués de mesure de paramètres physiologiques, par exemple, de rythme cardiaque ou de température, d'un individu ont été développés depuis quelques années afin de suivre l'évolution de ces paramètres notamment à des fins de surveillance de l'état de santé d'un individu.

Les fluides corporels tels que la sueur, la salive, les larmes, l'urine, le sang, le sérum, le plasma ou encore le liquide interstitiel contiennent des molécules chimiques, biochimiques ou biologiques qui renseignent sur l'état de santé d'un individu. Des ions, des protéines, des enzymes et des hormones sont, par exemple, présentes dans les urines. Leur concentration peut varier en fonction notamment de la prise de médicament, de paramètres environnementaux et des aliments ingérés par l'individu.

En particulier, l'analyse physico-chimique de la sueur apporte de nombreuses informations sur l'état d'un individu et peut être utilisée pour diagnostiquer des maladies, détecter des empoissonnements ou des allergies. Par exemple, la présence de xénobiotiques comme des pesticides, des polluants, des médicaments ou de la drogue peut être détectée par des électrodes sélectives. Ces électrodes de type ISE ("ion selective electrode", ISE) réagissent sélectivement à des ions spécifiques grâce à une couche sensible éventuellement fonctionnalisée par une sonde capable de complexer l'ion spécifique. De même, la mucoviscidose peut être diagnostiquée chez le nourrisson par mesure du taux de chlore dans la sueur. La variation de la conductivité ionique ou de la résistance électrique de la sueur, fonction de la concentration des ions, apporte des informations sur les modifications physiologiques d'un individu. La déshydratation d'un sportif ou d'un militaire peut, par exemple, être suivie à partir de la variation de la conductivité ionique de la sueur proportionnelle à l'osmolarité. Actuellement, le fluide corporel à analyser est collecté au préalable sur l'individu, par exemple grâce à des membranes absorbantes en forme de patch pour la sueur, puis analysé ultérieurement en laboratoire ou par l'individu lui-même. En effet, certains dispositifs d'analyse de fluide corporel peuvent être utilisé directement par l'individu lui-même et permettent ainsi de s'affranchir de la présence de personnel médical.

En particulier, le document WO99/35487 décrit, un dispositif d'analyse de fluide corporel pour la mesure électrochimique ou spectrophotométrique de la concentration d'analytes dans un fluide corporel par exemple le sang, la salive, l'urine et les fluides interstitiels. Le dispositif se présente sous la forme d'une bande de test comportant un substrat avec plusieurs compartiments contenant le fluide corporel à analyser et au moins un compartiment pour une solution de référence. Le fluide corporel et la solution de référence sont soumis à des conditions environnementales identiques et la valeur obtenue en temps réel est ajustée par rapport à celle de la solution de référence. Plus particulièrement, un dispositif de bande de test électrochimique ayant deux compartiments séparés permet de doser le taux de glucose dans le sang par une mesure différentielle entre la solution à analyser et une solution de référence de concentration connue. Le taux de glucose est, par exemple, déterminé à partir de peroxyde d'hydrogène dégagé lors de la réaction du glucose avec divers réactifs. Une sonde comme le ferrocène interagit avec le peroxyde d'hydrogène pour produire un courant proportionnel au taux de glucose. Ce dispositif permet une mesure ponctuelle précise d'un paramètre physico-chimique d'un liquide corporel mais n'est pas adapté à une mesure en continu permettant le suivi de l'évolution d'une pathologie ou d'un phénomène physiologique.

### Objet de l'invention

L'invention a pour but de proposer un dispositif d'analyse d'un fluide corporel remédiant aux inconvénients de l'art antérieur.

En particulier, l'invention a pour but de proposer un dispositif d'analyse d'un fluide corporel permettant de réaliser des mesures précises de paramètres physico-chimiques d'un fluide corporel pour suivre et contrôler l'état physiologique d'un individu.

L'invention a également pour but de proposer un dispositif d'analyse embarqué permettant de réaliser des mesures directement sur un individu, en temps réel et en continu.

Selon l'invention, ce but est atteint par un dispositif d'analyse selon les revendications indexées.

### Description sommaire des dessins

D'autres avantages et caractéristiques ressortiront plus clairement de la description qui va suivre des modes particuliers de réalisation de l'invention donnés à titre d'exemples non limitatifs et représentés aux dessins annexés, dans lesquels :
- La figure 1 représente, schématiquement et en coupe, un mode particulier de réalisation d'un dispositif d'analyse d'un fluide corporel selon l'invention.
- La figure 2 représente, schématiquement et en coupe, un autre mode particulier de réalisation d'un dispositif d'analyse d'un fluide corporel selon l'invention.
- La figure 3 représente, schématiquement en en vue de dessus, un autre mode particulier de réalisation du dispositif selon l'invention.
- La figure 4 représente trois séries de mesures effectuées avec un conductimètre à partir d'un dispositif, selon l'invention, utilisé pour l'analyse de la sueur d'un individu.

### Description des modes de réalisation particuliers de l'invention

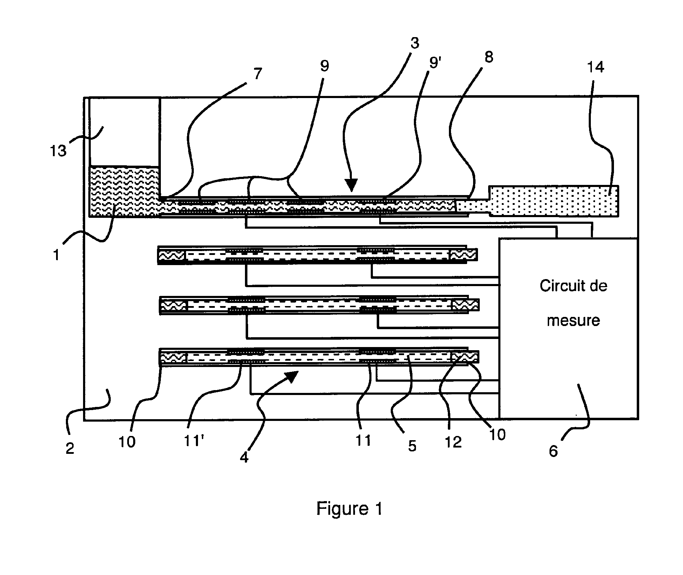

Selon un mode de réalisation particulier représenté à la figure 1, le dispositif d'analyse d'un fluide corporel 1 comporte un support 2, une cellule de mesure 3 contenant le fluide corporel 1 à analyser et au moins une cellule de référence 4 contenant un étalon 5. Le support 2 est muni d'au moins la cellule de mesure 3. Comme représenté à la figure 1, le support 2 est muni, de préférence, d'une cellule de mesure 3 et des cellules de référence 4 de sorte que les cellules 3 et 4 sont fixées au support 2. Le support 2 est, notamment, destiné à positionner la cellule de mesure 3 par rapport aux cellules de référence 4 et de maintenir en place les cellules de mesure et de référence, respectivement 3 et 4. Le dispositif d'analyse est, de préférence, un dispositif embarqué c'est-à-dire un dispositif portable dédié à l'analyse du fluide corporel 1 et comportant un circuit électronique de mesure 6. Le dispositif de mesure 6 peut être sur le support 2 (figure 1) ou éventuellement à l'extérieur du support 2.

La cellule de mesure 3 a avantageusement une structure capillaire et renferme un volume de fluide corporel 1 déterminé. Par structure capillaire, on entend une structure microtubulaire creuse où peut se produire un déplacement de fluide par capillarité. La cellule de mesure 3 possède un orifice d'entrée 7 et un orifice de sortie 8 du fluide corporel 1 et est munie d'au moins une électrode de travail de la cellule de mesure 9 et d'au moins une électrode de référence de la cellule de mesure 9'. Les électrodes de travail et de référence, de cellule de mesure, respectivement 9 et 9', sont en contact avec le fluide corporel 1 et disposées à distance l'une de l'autre le long de la cellule de mesure 3. Sur la figure 1, chaque électrode de mesure et de référence de la cellule de mesure, respectivement 9 et 9', est annulaire.

Selon une variante, l'électrode de travail de la cellule de mesure 9 et l'électrode de référence de la cellule de mesure 9' peuvent avoir d'autres géométries, par exemple, cylindriques, surfaciques en forme de disque, de parallélépipède ou de polygone.

Selon un mode de réalisation préférentiel, la cellule de référence 4 a des dimensions identiques à celles de la cellule de mesure 3. De même, chaque cellule de référence 4 peut être identique à la cellule de mesure 3, notamment en ce qui concerne le ou les matériaux constituant la cellule de référence 4 et sa structure, de préférence, capillaire.

Selon une variante, la cellule de référence 4 est homothétique de la cellule de mesure 3. La géométrie de la cellule de référence 4 a une forme similaire à celle de la cellule de mesure 3. Les angles et l'alignement des points de géométrie sont conservés mais les distances entre les points sont plus grandes ou plus petites.

La cellule de référence 4 renferme l'étalon 5. L'étalon 5 peut être une solution étalon liquide, un gel ou un solide. À titre d'exemple, l'étalon 5 peut être un matériau polymère contenant des ions en concentration connue ou un matériau à base de silice, un nitrure de silicium ou un matériau métallique ayant une résistance électrique connue. L'étalon 5 peut également être formé de plusieurs couches de matériaux différents, isolants et conducteurs, ayant une résistance et/ou une capacité connue(s). La cellule de référence 4 possède deux orifices 10 obturés et est munie d'au moins une électrode de travail de la cellule de référence 11 et d'au moins une électrode de référence de la cellule de référence 11'. L'électrode de travail et de référence de la cellule de référence, respectivement 11 et 11', sont en contact avec l'étalon 5. Au moins un des orifices 10 de la cellule de référence 4 est, avantageusement, obturé par un bouchon amovible 12. L'étalon 5 peut être ainsi changé ou renouvelé sans devoir enlever la cellule de référence 4 du dispositif embarqué d'analyse.

La cellule de référence 4 a au moins une électrode de travail de la cellule de référence 11 constituée par le ou les mêmes matériaux qu'une électrode de travail de la cellule de mesure 9. De même, la cellule de référence 4 a au moins une électrode de référence de la cellule de référence 11' constituée par le ou les mêmes matériaux qu'une électrode de référence de la cellule de mesure 9'.

Selon un mode de réalisation particulier, le dispositif embarqué d'analyse possède plusieurs cellules de référence 4 contenant chacune un étalon 5 sous forme d'une solution contenant une concentration connue d'une espèce, d'un analyte ou d'une molécule. La mesure simultanée ou consécutive, par exemple, de la conductivité de chaque solution étalon 5 et du fluide corporel 1 permet d'élaborer une courbe étalon corrélant la concentration de l'espèce dans les solutions étalons et la conductivité. Puis, la valeur de la concentration de l'espèce dans le fluide corporel 1 contenu dans la cellule de mesure 3 est déterminée à partir du résultat de conductivité du fluide corporel 1 et des courbes étalons.

Le dispositif peut également permettre de détecter la présence ou l'absence d'une espèce contenue dans le fluide corporel 1, telle qu'une bactérie, une protéine, du sucre, un xénobiotique ou un anticorps. Les cellules de mesure et de référence, respectivement 3 et 4, comportent alors au moins une électrode qui réagit sélectivement à des ions spécifiques de type électrode ionique sélective ("ion selective electrode", ISE). Cette électrode ISE est typiquement recouverte d'une couche sensible fonctionnalisée par une sonde spécifique de l'espèce à détecter, par exemple, des acides nucléiques de type ADN naturel ou synthétique, des protéines ou des enzymes.

Selon un mode de réalisation particulier, au moins une électrode de travail de la cellule de mesure 9 et au moins une l'électrode de travail de la cellule de référence 11 est une électrode de type ISE ("ion selective electrode", ISE) qui réagit sélectivement à des ions spécifiques. La reconnaissance de l'espèce cible entraîne alors une modification du signal électrique qui témoigne de la présence ou de l'absence de cette espèce cible.

Selon une variante, les cellules de mesure et de référence, respectivement 3 et 4, peuvent comporter plus de deux électrodes, par exemple, trois électrodes, avec une électrode de travail, une de référence et une contre-électrode ou encore un réseau d'électrodes avec une électrode de référence et plusieurs électrodes sélectives ISE, chacune sensible à une espèce cible spécifique.

Selon un autre mode de réalisation particulier, les électrodes de travail et de référence de la cellule de mesure, respectivement 9 et 9' et les électrodes de travail et de référence de la cellule de référence, respectivement 11 et 11', sont des électrodes métalliques.

A titre d'exemple, le dispositif d'analyse d'un fluide corporel, représenté à la figure 1, est constitué d'une cellule de mesure 3 et de trois cellules de référence 4. La cellule de mesure 3 est munie de quatre électrodes, une électrode de référence de la cellule de mesure 9' et trois électrodes de travail de la cellule de mesure 9, de préférence ISE, chacune étant formée par un matériau différent et destinée à la mesure d'une espèce cible spécifique. Chaque cellule de référence 4 est munie d'une électrode de référence de la cellule de référence 11' constituée par le ou les mêmes matériaux que l'électrode de référence de la cellule de mesure 9' et d'une électrode de travail de la cellule de référence 11 constituée par le même matériau qu'une des trois électrodes de travail de la cellule de mesure 9. Ainsi, chaque cellule de référence 4 mesure une espèce cible spécifique de l'étalon 5. La cellule de mesure 3 permet de réaliser les mesures des trois espèces cibles spécifiques dans le fluide corporel 1.

Selon une variante non représentée, le dispositif d'analyse peut être constitué d'une seule cellule de référence 4 et de plusieurs cellules de mesure 3. Chaque cellule de mesure 3 est alors munie d'une seule électrode de travail de la cellule de mesure 9 pour mesurer une seule espèce cible spécifique dans le fluide corporel 1. La cellule de référence 4 possède plusieurs électrodes de travail de la cellule de référence 11, chacune pour la mesure d'une espèce cible spécifique de l'étalon 5.

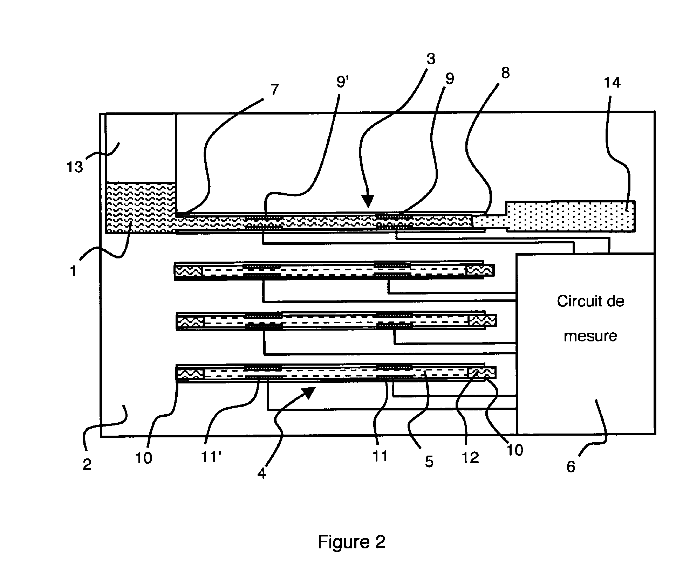

Selon un mode de réalisation préférentiel représenté à la figure 2, l'électrode de travail de la cellule de référence 11 et l'électrode de référence de la cellule de référence 11' sont constituées par le ou les mêmes matériaux que, respectivement, l'électrode de travail de la cellule de mesure 9 et l'électrode de référence de la cellule de mesure 9'. De plus, l'électrode de travail de la cellule de référence 11 et l'électrode de référence de la cellule de référence 11' sont identiques, respectivement, à l'électrode de travail de la cellule de mesure 9 et à l'électrode de référence de la cellule de mesure 9', en taille, en forme, en nombre et en position. L'étalon 5 est alors soumis aux mêmes conditions que celles du fluide corporel 1.

Les électrodes de travail et de référence de la cellule de mesure, respectivement, 9 et 9', et les électrodes de travail et de référence de la cellule de référence, respectivement, 11 et 11', sont connectées au circuit électronique de mesure 6 afin de réaliser des mesures électriques et/ou électrochimiques du fluide corporel 1 et de l'étalon 5. Pour calibrer les mesures et s'affranchir des variations dues aux paramètres environnementaux affectant la mesure, une mesure différentielle est réalisée entre la cellule de mesure 3 contenant le fluide corporel 1 et la cellule de référence 4 contenant l'étalon 5. Les valeurs obtenues pour le fluide corporel 1 sont réajustées en fonction de celles mesurées simultanément ou consécutivement pour l'étalon 5.

Les mesures peuvent être, par exemple, des mesures de conductivité électrique, de capacité, ampérométrique, chonoampérométrique potentiométrique, voltampérométrique et également des mesures de potentiel d'abandon. Le support 2 peut comporter un ou plusieurs microcontrôleurs capables de stocker les données et un ou plusieurs émetteur-récepteurs sans fil pour communiquer, par exemple par radiofréquence ou infrarouge, avec le circuit électronique de mesure 6 qui peut être à distance du support 2. L'information recherchée, par exemple la concentration d'une espèce dans le fluide corporel 1, peut être directement affichée à partir du traitement des valeurs de courant, de tension, de résistance ou d'impédance mesurées. L'information, directement affichée, peut également être la présence ou l'absence d'une pathologie ou d'une espèce cible telle qu'une protéine.

Les mesures électriques ou électrochimiques sont sensibles à l'environnement dans lequel elles sont réalisées. Dans des conditions de mesures réelles, notamment lorsque les mesures sont effectuées directement sur un individu, le changement de l'environnement dans lequel évolue l'individu affecte les mesures électriques ou électrochimiques. Elles sont particulièrement sensibles à des paramètres environnementaux tels que la température, l'hygrométrie ou encore la nature des matériaux des électrodes utilisées. De même, la forme des électrodes et la distance entre les électrodes font varier les mesures réalisées. Pour s'affranchir de ces paramètres environnementaux, les mesures électriques ou électrochimiques sont réalisées à partir du fluide corporel 1 contenu dans la cellule de mesure 3 et, simultanément ou consécutivement, sur l'étalon 5 contenu dans la cellule de référence 4. L'utilisation d'une ou de plusieurs cellules de référence 4 permet de réaliser des mesures comparatives entre au moins un étalon 5, dont on connaît les caractéristiques chimiques et/ou physiques et le fluide corporel 1 pour lequel la mesure est réalisée.

Par ailleurs, pour une même mesure électrique et électrochimique, une dérive dans le temps est souvent observée et implique généralement la calibration du dispositif de mesure ou d'analyse avant chaque mesure. La mesure différentielle entre la cellule de mesure 3 et la cellule de référence 4 permet également de s'affranchir de cette dérive. Cette caractéristique est particulièrement intéressante dans le cas de mesures, en continu, échelonnées dans le temps.

La cellule de mesure 3 et la cellule de référence 4 sont, avantageusement, des tubes capillaires constitués d'un matériau choisi parmi du verre, de la silice fondue et un matériau polymère. Les tubes capillaires en verre ou silice fondue ont un diamètre intérieur compris, de préférence, entre 20 µm et 700µm et les tubes capillaires en matériau polymère entre 100 µm et 1500µm. La longueur du tube capillaire est, de préférence, comprise entre quelques mm et 20 cm, plus particulièrement, entre 5mm et 5 cm.

La cellule de mesure 3 ainsi que la cellule de référence 4 peuvent, avantageusement, être fixées, collées ou intégrées à un même support 2. Le support 2 est, de préférence, constitué par un matériau souple et inerte vis-à-vis du fluide corporel 1 et de l'étalon 5. Il est, de préférence, choisi parmi un textile synthétique, naturel ou artificiel, un matériau polymère et un matériau semi-conducteur. À titre d'exemple, le support 2 est un tissu tel qu'un vêtement, une compresse ou un bandage, sur lequel sont collées la cellule de mesure 3 ainsi que la ou les cellules de référence 4. Le support 2 peut également être un matériau semi-conducteur rigide tel qu'une plaque de silicium ou de germanium suffisamment amincie pour acquérir la souplesse nécessaire à sa fixation ou son intégration dans un support souple.

Selon un mode de réalisation préférentiel, le support 2 est un tissu inerte vis-à-vis du fluide corporel 1 et de l'étalon 5. La cellule de mesure 3 et la cellule de référence 4 font partie intégrante du support 2. La structure capillaire des cellules de mesure et de référence, respectivement 3 et 4, est formée, par exemple, par le tissage du support 2.

Le fluide corporel 1 à analyser peut être collecté directement sur l'individu puis analysé sans traitement préalable, par exemple, pour la sueur ou la salive. Dans ce cas, le dispositif peut, avantageusement, être porté sur l'individu. En particulier, le dispositif embarqué d'analyse peut être en contact avec la peau de l'individu pour analyser de la sueur.

Le fluide corporel 1 peut également subir un traitement avant analyse. Du plasma est, par exemple, obtenu par centrifugation préalable du sang prélevé sur l'individu puis analysé de façon ponctuelle par le dispositif embarqué d'analyse qui n'est pas, dans ce cas, porté par l'individu ou en contact avec l'individu.

Chaque orifice d'entrée 7 et de sortie 8 de la cellule de mesure 3 est connecté à des moyens de drainage microfluidique qui forcent l'écoulement du fluide corporel 1 de l'orifice d'entrée 7 vers l'orifice de sortie 8. Les moyens de drainage microfluidique peuvent être soit actifs soit passifs. Les moyens de drainage microfluidique sont, de préférence, formés par un organe de collecte 13 du fluide corporel 1 connecté à l'orifice d'entrée 7 de la cellule de mesure 3 et une pompe microfluidique 14 reliée à l'orifice de sortie 8 de la cellule de mesure 3. L'action conjuguée de l'organe de collecte 13 et de la pompe microfluidique 14 crée un phénomène de drainage du fluide corporel 1 à l'intérieur de la cellule de mesure 3.

La pompe microfluidique 14 peut par exemple être une pompe à vide constituant des moyens de drainage microfluidique actifs. Elle peut également être formée par un tissu absorbant solidaire de la cellule de mesure 3 et bouchant l'orifice de sortie 8, constituant alors des moyens de drainage microfluidique passifs.

Selon un mode de réalisation particulier représenté à la figure 2, le dispositif d'analyse est en contact avec la peau d'un individu pour analyser sa sueur. L'organe de collecte 13 est constitué par une poche de collecte du fluide corporel 1 fixée au support 2. La poche de collecte permet de recueillir le fluide corporel 1 par une ouverture (non représentée) en contact avec la peau.

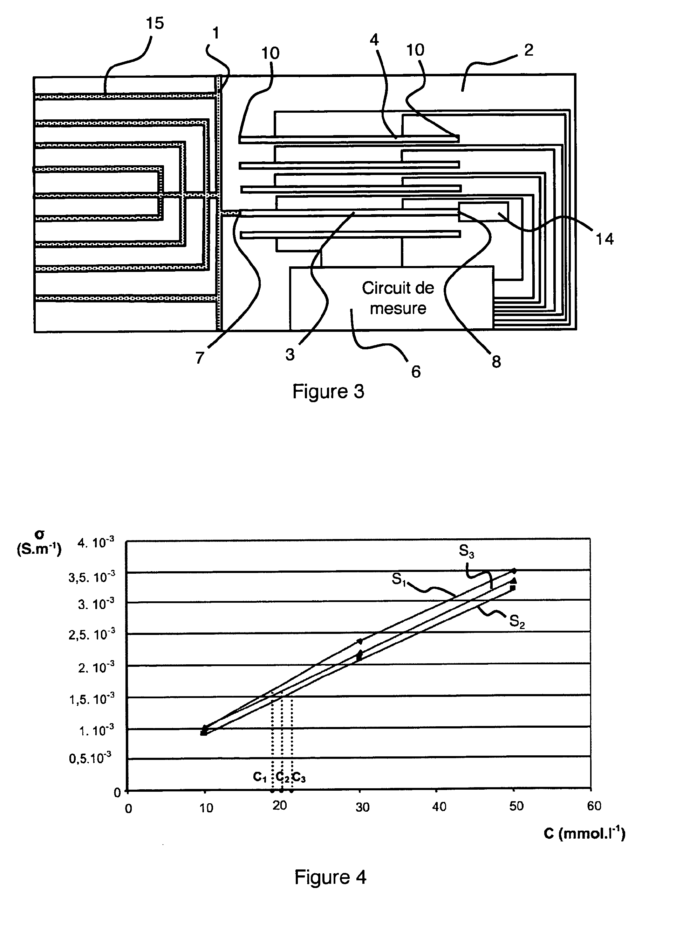

Selon une variante représentée à la figure 3, le dispositif d'analyse, en contact avec la peau d'un individu pour analyser sa sueur, comporte un organe de collecte 13 constitué par un réseau de canaux 15 intégré au support 2. Une partie ouverte des canaux 15 est alors disposée face à la peau de l'individu afin de récolter la sueur et la drainer vers la cellule de mesure 3. Le support 2 peut, par exemple, être un tissu constitué de parties hydrophiles en contact avec la peau et de parties hydrophobes. Les parties hydrophiles et hydrophobes sont agencées de façon à créer le réseau de canaux 15 qui achemine la sueur vers la cellule de mesure 3.

Selon une variante non représentée, l'organe de collecte est constitué par un tuyau relié par une de ses extrémités à la cellule de mesure 3 et par l'autre extrémité, directement à la source générant le fluide corporel 1. Cette source peut être une bouche pour collecter de la salive ou une aiguille pour collecter du sang.

À titre d'exemple, des mesures en conductimétrie sont réalisées sur un conductimètre CDM 210, commercialisé par la société Radiometer Analytical, à partir d'un dispositif embarqué d'analyse ayant un support 2 en tissu à base de polyimide, par exemple à base de Kapton® ou de polyéthylène, par exemple du polyéthylène téréphtalate (PET) ou encore de polychlorure de vinyle (PVC). Le dispositif embarqué comporte un organe de collecte 13 constitué d'une poche et une pompe microfluidique 14 basée sur la capillarité, constituée par un tissu absorbant. Le dispositif comporte également une cellule de mesure 3 et trois cellules de référence 4. La cellule de mesure 3 est munie de deux électrodes métalliques en Platine constituant l'électrode de travail et de référence, de la cellule de mesure, respectivement 9 et 9'. Chacune des cellules de référence 4 est munie de deux électrodes métalliques en Platine constituant l'électrode de travail et de référence, de la cellule de référence, respectivement 11 et 11'. Les mesures sont effectuées sur de la sueur collectée sur un individu et trois solutions étalon 5 de chlorure de sodium respectivement à une concentration molaire de 10mmol.l⁻¹, 30mmol.l⁻¹, 50mmol.l⁻¹.

Chacune des cellules de mesure et de référence, respectivement 3 et 4, est constituée par un tube de 1 mm de diamètre interne et l'électrode de travail de la cellule de mesure 9 et l'électrode de travail de la cellule de référence 11, ont une surface de mesure de 2 mm². Trois séries de mesures sont réalisées avec un intervalle entre chaque série de mesures d'environ 2 min.

Pour chaque série de mesures, la valeur de la conductivité est relevée pour les trois solutions étalons 5 et la sueur. Sur la figure 4, trois courbes étalons σ=f(C), représentant la conductivité des solutions mesurées par rapport à la concentration, sont ainsi obtenues. Les courbes S₁, S₂ et S₃ correspondent respectivement à la première, seconde et troisième série de mesures. À partir de ces courbes étalons, on peut déterminer trois concentrations ioniques de la sueur C₁, C₂ et C₃ respectivement d'une valeur de 19mmol.l⁻¹, 20mmol.l⁻¹ et 21mmol.l⁻¹. Ces trois valeurs peuvent être utilisées pour déterminer la concentration de la sueur, par exemple en prenant la moyenne de ces trois valeurs, ou pour suivre l'évolution de la concentration de la sueur en fonction du temps.

Contrairement aux procédés de l'art antérieur, le dispositif embarqué d'analyse de fluide corporel 1 permet un suivi en temps réel, continu et non invasif de paramètres physiologiques à partir de l'analyse d'un fluide corporel 1. Les mesures sont également obtenues par ce dispositif avec une grande précision.

Bien que les électrodes de travail de la cellule de mesure 9 et les électrodes de travail de la cellule de référence 11 représentées sur les figures soient en contact respectivement avec le fluide corporel 1 et avec l'étalon 5, l'invention n'est pas limitée à ce type d'électrodes. En particulier, dans le cas de mesures capacitives, les électrodes peuvent être disposées à l'extérieur des capillaires.

Par ailleurs, bien que les exemples donnés illustrent un dispositif comportant un support 2, l'invention n'est pas limitée à ce type de dispositif. L'invention couvre également un dispositif d'analyse dépourvu de support 2, le dispositif d'analyse étant ainsi formés de cellules de mesure et de référence, 3 et 4, agencées indépendamment les unes des autres.

## Revendications

1. Dispositif d'analyse d'un fluide corporel (1) comportant :
- une cellule de mesure (3) contenant le fluide corporel (1) à analyser et munie d'au moins une électrode de travail de la cellule de mesure (9) et d'au moins une électrode de référence de la cellule de mesure (9'),
- au moins une cellule de référence (4) contenant un étalon (5) et munie d'au moins une électrode de travail de la cellule de référence (11) et d'au moins une électrode de référence de la cellule de référence (11'), au moins une électrode de travail de la cellule de référence (11) étant constituée par le ou les mêmes matériaux qu'une électrode de travail de la cellule de mesure (9) et au moins une électrode de référence de la cellule de référence (11') étant constituée par le ou les mêmes matériaux qu'une électrode de référence de la cellule de mesure (9') et,
- un circuit électronique de mesure (6) connecté aux électrodes de travail et de référence, de la cellule de mesure (9, 9') et de la cellule de référence (11, 11'),
dispositif **caractérisé en ce que** la cellule de mesure (3) a un orifice d'entrée (7) et un orifice de sortie (8) du fluide corporel (1), chacun étant connecté à des moyens de drainage microfluidique (13, 14, 15), et **en ce que** la cellule de référence (4) possède deux orifices (10) obturés.

2. Dispositif selon la revendication 1, **caractérisé en ce qu'**il comporte un support (2), ledit support (2) étant muni d'au moins la cellule de mesure (3).

3. Dispositif selon l'une des revendications 1 et 2, **caractérisé en ce que** l'électrode de travail de la cellule de référence (11) et l'électrode de référence de la cellule de référence (11') sont identiques, respectivement, à l'électrode de travail de la cellule de mesure (9) et à l'électrode de référence de la cellule de mesure (9'), en taille, en forme, en nombre et en position.

4. Dispositif selon l'une quelconque des revendications 1 à 3, **caractérisé en ce que** la cellule de référence (4) a des dimensions identiques à celles de la cellule de mesure (3).

5. Dispositif selon l'une quelconque des revendications 1 à 4, **caractérisé en ce que** la cellule de mesure (3) et la cellule de référence (4) ont une structure capillaire.

6. Dispositif selon la revendication 5, **caractérisé en ce que** la cellule de mesure (3) et la cellule de référence (4) sont des tubes capillaires constitués d'un matériau choisi parmi du verre, de la silice fondue et un matériau polymère.

7. Dispositif selon l'une quelconque des revendications 1 à 6, **caractérisé en ce que** les moyens de drainage microfluidique sont formés par un organe de collecte (13) du fluide corporel (1) connecté à l'orifice d'entrée (7) de la cellule de mesure (3) et une pompe microfluidique (14) reliée à l'orifice de sortie (8) de la cellule de mesure (3).

8. Dispositif selon l'une quelconque des revendications 1 à 7, **caractérisé en ce que** le dispositif d'analyse est en contact avec la peau d'un individu pour analyser de la sueur et **en ce que** l'organe de collecte (13) est constitué par un réseau de canaux (15) intégré au support (2).

9. Dispositif selon l'une quelconque des revendications 1 à 7, **caractérisé en ce que** le dispositif d'analyse est en contact avec la peau d'un individu pour analyser de la sueur et **en ce que** l'organe de collecte (13) est constitué par une poche de collecte du fluide corporel fixée au support (2).

10. Dispositif selon l'une quelconque des revendications 7 à 9, **caractérisé en ce que** la pompe microfluidique (14) est formée par un tissu absorbant solidaire de la cellule de mesure (3) et bouchant l'orifice de sortie (8).

11. Dispositif selon l'une quelconque des revendications 1 à 10, **caractérisé en ce qu'**au moins un des orifices (10) de la cellule de référence (4) est obturé par un bouchon amovible (12).

12. Dispositif selon l'une quelconque des revendications 2 à 11, **caractérisé en ce que** le support (2) est constitué par un matériau souple.

13. Dispositif selon la revendication 12, **caractérisé en ce que** le support (2) est constitué par un matériau choisi parmi un textile synthétique, naturel ou artificiel, un matériau polymère et un matériau semi-conducteur.

14. Dispositif selon l'une quelconque des revendications 1 à 13, **caractérisé en ce que** les électrodes de travail et de référence de la cellule de mesure (9, 9') et les électrodes de travail et de référence, de la cellule de référence (11, 11') sont des électrodes métalliques.

15. Dispositif selon l'une quelconque des revendications 1 à 13, **caractérisé en ce qu'**au moins une électrode de travail de la cellule de mesure (9) et au moins une l'électrode de travail de la cellule de référence (11) est une électrode de type ISE ("ion selective electrode", ISE) qui réagit sélectivement à des ions spécifiques.

16. Dispositif selon l'une quelconque des revendications 1 à 15, **caractérisé en ce que** l'étalon (5) est une solution liquide ou un gel.

17. Dispositif selon l'une quelconque des revendications 1 à 15, **caractérisé en ce que** l'étalon (5) est un étalon solide.

## Patentansprüche

1. Vorrichtung zur Untersuchung einer Körperflüssigkeit (1), welche aufweist:
- eine Messzelle (3), die die zu untersuchende Körperflüssigkeit enthält und mit mindestens einer Arbeitselektrode (9) der Messzelle und mit mindestens einer Referenz-Elektrode (9') der Messzelle ausgestattet ist,
- mindestens eine Referenz-Zelle (4), die ein Eichmuster (5) enthält und mit mindestens einer Arbeitselektrode (11) der Referenz-Zelle und mit mindestens einer Referenz-Elektrode (11') der Referenz-Zelle ausgestattet ist, wobei mindestens eine Arbeitselektrode (11) der Referenz-Zelle aus dem oder den gleichen Werkstoff(en) gebildet ist wie eine Arbeitselektrode (9) der Messzelle, und mindestens eine Referenz-Elektrode (11') der Referenz-Zelle aus dem oder den gleichen Werkstoff(en) gebildet ist wie eine Referenz-Elektrode (9') der Messzelle, und
- einen elektronischen Messkreis (6), der mit den Arbeits- und Referenz-Elektroden (9, 9', 11, 11') der Messzelle und der Referenz-Zelle verbunden ist,
**dadurch gekennzeichnet,**
**dass** die Messzelle (3) eine Einlassöffnung (7) und eine Auslassöffnung (8) für die Körperflüssigkeit (1) hat, die jeweils an Drainungsmittel für Mikrofluide (13,14, 15) angeschlossen sind, und dass die Referenz-Zelle (4) zwei verschlossene Öffnungen (10) besitzt.

2. Vorrichtung nach Anspruch 1,
**dadurch gekennzeichnet,**
**dass** sie einen Halter (2) aufweist, wobei dieser Halter (2) mit mindestens einer Messzelle (3) ausgestattet ist.

3. Vorrichtung nach einem der Ansprüche 1 und 2,
**dadurch gekennzeichnet,**
**dass** die Arbeitselektrode (11) der Referenz-Zelle und die Referenz-Elektrode (11') der Referenz-Zelle der Arbeitselektrode (9) der Messzelle bzw. der Referenz-Elektrode (9') der Messzelle gleichen, was die Größe, die Form, die Anzahl und die Position betrifft.

4. Vorrichtung nach einem der Ansprüche 1 bis 3,
**dadurch gekennzeichnet,**
**dass** die Referenz-Zelle (4) die gleichen Abmessungen hat wie die Messzelle (3).

5. Vorrichtung nach einem der Ansprüche 1 bis 4,
**dadurch gekennzeichnet,**
**dass** die Messzelle (3) und die Referenz-Zelle (4) einen kapillarförmigen Aufbau aufweisen.

6. Vorrichtung nach Anspruch 5,
**dadurch gekennzeichnet,**
**dass** die Messzelle (3) und die Referenz-Zelle (4) Kapillarröhrchen sind, die aus einem Werkstoff bestehen, der aus Glas, Quarzgut und einem Polymerstoff ausgewählt ist.

7. Vorrichtung nach einem der Ansprüche 1 bis 6,
**dadurch gekennzeichnet,**
**dass** die Drainungsmittel für Mikrofluide von einer Sammeleinrichtung (13) für die Körperflüssigkeit (1), die mit der Einlassöffnung (7) der Messzelle (3) verbunden ist, und einer Mikrofluid-Pumpe (14), die mit der Auslassöffnung (8) der Messzelle (3) verbunden ist, gebildet werden.

8. Vorrichtung nach einem der Ansprüche 1 bis 7,
**dadurch gekennzeichnet,**
**dass** sich die Untersuchungsvorrichtung in Kontakt mit der Haut eines Körpers befindet, um Schweiß zu untersuchen, und dass die Sammeleinrichtung (13) von einem Kanalnetz (15) gebildet wird, das in den Halter (2) integriert ist.

9. Vorrichtung nach einem der Ansprüche 1 bis 7,
**dadurch gekennzeichnet,**
**dass** sich die Untersuchungsvorrichtung in Kontakt mit der Haut eines Körpers befindet, um Schweiß zu untersuchen, und dass die Sammeleinrichtung (13) von einem Sammelbeutel für die Körperflüssigkeit gebildet wird, der an dem Halter (2) befestigt ist.

10. Vorrichtung nach einem der Ansprüche 7 bis 9,
**dadurch gekennzeichnet,**
**dass** die Mikrofluid-Pumpe (14) von einem absorbierenden Gewebe gebildet wird, das fest mit der Messzelle (3) verbunden ist und in die Auslassöffnung (8) mündet.

11. Vorrichtung nach einem der Ansprüche 1 bis 10,
**dadurch gekennzeichnet,**
**dass** wenigstens eine der Öffnungen (10) der Referenz-Zelle (4) durch einen abnehmbaren Deckel (12) verschlossen ist.

12. Vorrichtung nach einem der Ansprüche 2 bis 11,
**dadurch gekennzeichnet,**
**dass** der Halter (2) aus einem biegsamen Werkstoff gebildet ist.

13. Vorrichtung nach Anspruch 12,
**dadurch gekennzeichnet,**
**dass** der Halter (2) aus einem Werkstoff gebildet ist, der aus einem synthetischen, natürlichen oder künstlichen Textil, einem Polymerstoff und einem Halbleiter-Material gewählt wird.

14. Vorrichtung nach einem der Ansprüche 1 bis 13,
**dadurch gekennzeichnet,**
**dass** die Arbeits- und die Referenz-Elektrode (9, 9') der Messzelle sowie die Arbeits- und die Referenz-Elektrode (11, 11') der Referenz-Zelle metallene Elektrode sind.

15. Vorrichtung nach einem der Ansprüche 1 bis 13,
**dadurch gekennzeichnet,**
**dass** mindestens eine Arbeitselektrode (9) der Messzelle und mindestens eine Arbeitselektrode (11) der Referenz-Zelle eine Elektrode des Typs ISE ("ion selective electrode", ISE) ist, die selektiv auf spezifische Ionen reagiert.

16. Vorrichtung nach einem der Ansprüche 1 bis 15,
**dadurch gekennzeichnet,**
**dass** das Eichmuster (5) eine flüssige Lösung oder ein Gel ist.

17. Vorrichtung nach einem der Ansprüche 1 bis 15,
**dadurch gekennzeichnet,**
**dass** das Eichmuster (5) ein Festkörper ist.

## Claims

1. A bodily fluid analysis device (1) comprising:
• a measuring cell (3) containing the bodily fluid (1) to be analyzed and provided with at least one work electrode of the measuring cell (9) and at least one reference electrode of the measuring cell (9'),
• at least one reference cell (4) containing a reference standard (5) and provided with at least one work electrode of the reference cell (11) and at least one reference electrode of the reference cell and (11'), at least one work electrode of the reference cell (11) being formed by the same material or materials as a work electrode of the measuring cell (9) and at least one reference electrode of the reference cell (11') being formed by the same material or materials as a reference electrode of the measuring cell (9') and,
• an electronic measuring circuit (6) connected to the work and reference electrodes of the measuring cell (9, 9') and of the reference cell (11,11'),
device **characterized in that** the measuring cell (3) has an inlet orifice (7) and an outlet orifice (8) of the bodily fluid (1), each orifice being connected to microfluidic draining means (13, 14, 15), and **in that** the reference cell (4) has two sealed-off orifices (10).

2. The device according to claim 1, comprising a support (2), said support (2) being provided with at least the measuring cell (3).

3. The device according to either claim 1 or 2, **characterized in that** the work electrode of the reference cell (11) and the reference electrode of the reference cell (11') are respectively identical to the work electrode of the measuring cell (9) and to the reference electrode of the measuring cell (9') in size, shape, number and position.

4. The device according to any one of claims 1 to 3, **characterized in that** the reference cell (4) has identical dimensions to those of the measuring cell (3).

5. The device according to any one of claims 1 to 4, **characterized in that** the measuring cell (3) and the reference cell (4) have a capillary structure.

6. The device according to claim 5, **characterized in that** the measuring cell (3) and the reference cell (4) are capillary tubes made from a material chosen from glass, molten silica and a polymer material.

7. The device according to any one of claims 1 to 6, **characterized in that** the microfluidic draining means are formed by collecting means (13) of the bodily fluid (1) connected to the inlet orifice (7) of the measuring cell (3) and a microfluidic pump (14) connected to the outlet orifice (8) of the measuring cell (3).

8. The device according to any one of claims 1 to 7, **characterized in that** the analysis device is in contact with a person's skin to analyze sweat, and **in that** the collecting means (13) consist of a network of channels (15) integrated in the support (2).

9. The device according to any one of claims 1 to 7, **characterized in that** the analysis device is in contact with a person's skin to analyze sweat, and the collecting means (13) consist of a collect pocket of the bodily fluid fixed to the support (2).

10. The device according to any one of claims 7 to 9, **characterized in that** the microfluidic pump (14) is formed by an absorbent fabric integral to the measuring cell (3) and sealing off the outlet orifice (8).

11. The device according to any one of claims 1 to 10, **characterized in that** at least one of the orifices (10) of the reference cell (4) is sealed off by a removable plug (12).

12. The device according to any one of claims 2 to 11, **characterized in that** the support is formed by a flexible material.

13. The device according to claim 12 **characterized in that** the support (2) is formed by a material chosen from a synthetic, natural or artificial textile, a polymer material and a semiconducting material.

14. The device according to any one of claims 1 to 13, **characterized in that** the working and reference electrodes of the measuring cell (9, 9') and the working and reference electrodes of the reference cell (11, 11') are metallic electrodes.

15. The device according to any one of claims 1 to 13, **characterized in that** at least one working electrode of the measuring cell (9) and at least one working electrode of the reference cell (11) is an ion selective electrode (ISE) which reacts selectively to specific ions.

16. The device according to any one of claims 1 to 15, **characterized in that** the reference standard (5) is a liquid solution or a gel.

17. The device according to any one of claims 1 to 15, **characterized in that** the reference standard (5) is a solid standard.
